# EUROPEAN PATENT APPLICATION

(11) **EP 2 716 311 A1**
(43) Date of publication of application: **09.04.2014**
(21) Application number: 13382364.1
(22) Date of filing: 19.09.2013
(51) Int. Cl.: A61M 3/02

(54) **Disposable device for internal anal canal hygiene**

(30) Priority: 19.09.2012 ES 201200874 U; 19.09.2012 ES 201201155; 27.10.2012 ES 201201094; 02.11.2012 ES 201201126
(71) Applicant: Hernandez Mondejar, Jose, 30205 Cartagena (Murcia) (ES)
(72) Inventor: Hernandez Mondejar, Jose, 30205 Cartagena (Murcia) (ES)
(74) Representative: Carpintero Lopez, Francisco

(57) **Abstract**

The present invention refers to a disposable device for internal anal canal hygiene, comprising an elongated cannula (2) connected to a deposit (1) that contains a physiological solution, wherein the cannula (2) is provided with at least one orifice (6) in fluid communication with the deposit (1) and, wherein the deposit (1) is formed of a depressible material for pumping said physiological solution to the cannula (2) when a pressure is exerted. Preferably, the cannula (2) has a free end (4) finished with a rounded tip (5) wherein the orifices (6), configured at its lateral sides, can be protected by a seal. Preferably, the physiological solution is composed of a physiological fluid to drag the rest of feces of the walls of the anal canal, such as physiological saline or purified water. Said physiological solution may further comprise other components with organoleptic or medicinal properties.

## Description

### Object of the invention

The present invention relates to a disposable device refillable with a physiological solution, which is applied to the hygiene of the internal anal canal, preferably after defecation, to remove residual feces. The device offers cleanliness and freshness in the area and improves the well-being feeling of the user.

### Background of the invention

In order to solve obstinate constipation, there are products existing today intended for rectal stimulation before defecation (enemas). There are also systems for cleaning the digestive tract intended for preparing the bowel in order to perform a medical procedure (diagnostic test or surgical treatment). There are also non-disposable irrigation systems for daily cleaning of the external anal region by means of using running water from a home installation.

The disposable or non-disposable containers intended for performing enemas have a cannula that is longer or shorter in length, with an orifice in its upper part, such that the liquid it contains flows out in the same direction the cannula is pointing, therefore reaching the rectum, given that its function is to induce defecation in the event of constipation or to clean the colon for performing medical procedures.

Meanwhile, the cleaning of the anal region by means of disposable systems after defecation is limited to the external perianal part, using paper, wet wipes or water baths. The inner part of the anus cannot be cleaned since this region cannot be reached with the described methods.

This impossibility of post-defecation internal cleaning sometimes even prevents complete external cleaning due to the matter moving outwards little by little, with the usual discomforts caused by soiling, smell, irritation...

Meanwhile, the presence of decomposing fecal matter inside the anus can affect a person's health condition, causing different types of infections and pathologies (hemorrhoids, fistulas, fissures...) or worsening them.

An easy-to-handle cleaning device the objective of which is to clean internal anal canal without reaching the rectum, and therefore without stimulating defecation, and which can be used daily in post-defecation anal hygiene, offering suitable results in terms of hygiene and comfort is not available.

### Description of the invention

This invention overcomes the drawbacks above mentioned by providing a disposable device for internal anal canal hygiene, which achieves cleaning the region without stimulating defecation.

In one aspect of the invention, the disposable device for internal anal canal hygiene, comprises an elongated cannula connected to a deposit refillable with a physiological solution, wherein the cannula is provided with at least one orifice in fluid communication with the deposit and, wherein the deposit is formed of a depressible material for pumping said physiological solution to the cannula when pressure is exerted in said deposit.

The invention proposes a disposable, hygienic cleaning device for use anytime to clean the internal anal canal preferably after defecation. For achieving these purposes and as above-mentioned, the cannula is long enough to be inserted through the anus without reaching the rectum, in order to prevent stimulating defecation.

Preferably, the device essentially consists of a deposit made of plastic, aluminum or another depressible material, having a diverse capacity, with a pear shape, tube shape or another shape having similar features, ending in a flexible or rigid cannula for insertion through the anal orifice and reaching only the internal anal canal.

The cannula preferably ends in a rounded blind tip facilitating its insertion through the external anal sphincter. It will preferably have one or several side orifices allowing the irrigation of the internal anal canal with the liquid used for anal cleaning such that the walls of the internal anal canal are reached without the liquid reaching the rectum.

Preferably, the cannula which is connected to the deposit is sealed such that the leak-tightness of the deposit is assured. However, according to another preferred embodiment, the cannula can be separated from and connected to the deposit at the time of use by various systems, be it by screwing, by pressure or by another similar system.

The refillable deposit can be filled with a physiological solution which will be used for internal cleaning of the anus and which will remove the fecal matter adhered to the walls of the internal anal canal after defecation. The physiological solution may be refreshing, giving the user a sense of freshness and cleanliness in the region.

Likewise, the deposit can be filled with a physiological solution intended for internal anal canal hygiene to which different components can be added, such as organoleptic components or active principles for anal pathologies treatment, so that they provide a greater sense of cleanliness, freshness and well-being to the user or for relieving or curing different pathologies.

The main ingredient of this physiological solution is a physiological entraining liquid, such as physiological saline, purified water or any other liquid, which performs the function of cleaning and entraining fecal matter from the walls of the internal anal canal. Said physiological entraining liquid can be used to dissolve possible additional components.

Menthol can preferably be added to the physiological solution due to its antiinflammatory, anti-pruritic, weak antiseptic properties and to the sense of freshness it provides. Likewise, the physiological solution can comprise components serving as a vehicle so that those components which are added to the physiological solution for increasing the sense of cleanliness, freshness and well-being can be solubilized in the physiological solution. This is the case of menthol which cannot be directly dissolved in water or in physiological saline, such that by using glycerin as a vehicle thereof, the menthol solubilizes in glycerin, and this solution then solubilizes in physiological saline or purified water.

Furthermore, components providing the physiological solution with organoleptic properties which increase the sense of user well-being, for example several drops of mint, or strawberry, or rose essence, can be added.

Active ingredients for specific anal pathology treatment can also be added.

Many discomforts derived from permanent build-up of fecal matter in the -anus are prevented with the proposed physiological solution for cleaning the internal anal canal, such discomforts including a bad smell, itching or stinging sensation in the anus, soiling of the anus and of the article of clothing in contact with the anus, and among others, the bad smell when passing intestinal gas.

Furthermore, it aids in reducing the proliferation of anal infections, particularly if the user suffers a anal pathology (both internal and external hemorrhoids, fistulas, fissures...), while at the same time the cleaning of the internal anal canal aids in improving the absorption of medication administered by anal route (anti-hemorrhoid creams, suppositories...).

It also provides a sense of well-being, both due to the sense of cleanliness and to the properties of the different components that can be added to the physiological solution, be it an aroma or freshness, such as the case of menthol.

### Brief description of the drawings

For a better comprehension of the invention the following drawings are provided for illustrative and non limiting purposes, wherein:
Figure 1 shows an embodiment of the disposable device for internal anal canal hygiene.
Figure 2 shows another embodiment of the disposable device for internal anal canal hygiene.

### Preferred embodiments of the invention

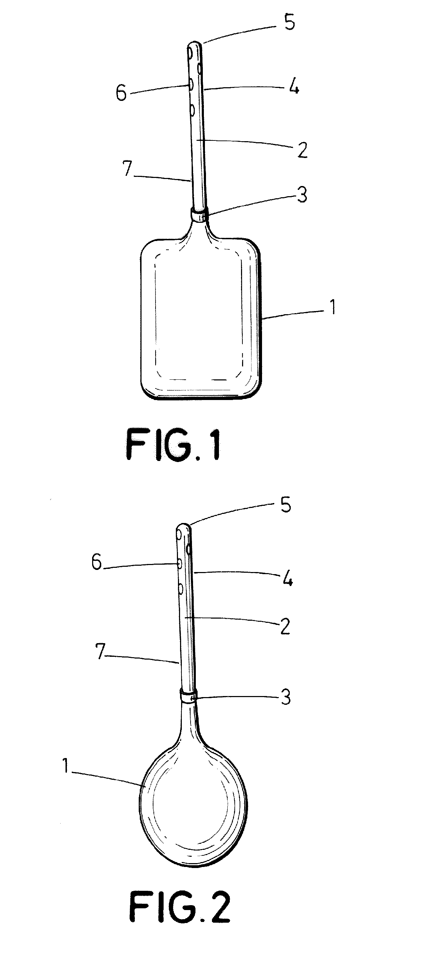

In the preferred embodiments of figures 1 and 2, it is shown two possible embodiments of the single-use disposable device for internal anal canal hygiene, which comprises a refillable deposit 1, which is connected to a flexible or rigid cannula 2, wherein said cannula 2 is provided with at least one orifice 6 that is in fluid communication with the deposit 1. The deposit 1 is formed of a depressible material, for pumping said physiological solution to the cannula 2 when pressure is exerted in said deposit 1. The deposit 1 can have a pear shape, tube shape or a similar shape.

According to a preferred embodiment, the cannula 2 and the deposit 1 are joined together forming a one single piece device. According to another preferred embodiment, the cannula 2 is connected to the deposit 1 by means of screwed, pressure, piercing or threading consisting of a two-piece device.

Preferentially, the cannula 2 has two ends, a proximal end 7, through which the cannula 2 is connected to the deposit 1 and, a free end 4, opposite to the proximal end 7.

The cannula 2 is long enough to be inserted through the anus such that its free end 4 is completely inserted into the internal anal canal. This free end 4 of the cannula 2 is provided with a rounded tip 5, preferably blind, and one or several side orifices 6 where the solution for cleaning the internal anal canal flows out.

According to a preferred embodiment, the cannula 2 comprises a bellows system 3 at its proximal end 7. According to another preferred embodiment, the deposit 1 comprises a bellows system 3 adjacent to its connection with the cannula 2. In both embodiments, the bellows system 3 facilitates the curvature for inserting the cannula 2 in the anus. Preferably, the connection between the deposit 1 and the cannula 2 corresponds with the bellows system 3, wherein said connection may be made by any connection system, such as threading, pressure...

According to another preferred embodiment, the device may further comprise a seal to protect the perforated area of the free end 4 of the cannula 2.

Preferably, the refillable deposit 1 is filled with a physiological solution composed of a physiological entraining liquid to drag the rest of feces of the walls of the internal anal canal , such as physiological saline or purified water. When the user pressed the deposit 1 with the hand, the physiological solution gets out through the cannula 2 in sufficient quantity to exert pressure on the walls of the internal anal canal and cause natural evacuation of the liquid along with the remains of feces that were left in the internal anal canal, leaving the area clean and fresh feeling.

According to another preferred embodiment, the physiological solution further comprises menthol to provide freshness. Likewise, the physiological solution may further comprise organoleptic components, such as mint, strawberry, or rose essence, and/or, active principles for anal pathologies treatment. Additionally, the physiological solution may further comprise a vehicle component to solubilize the physiological solution, such as glycerin.

Thus, the device would preferably be used in the following manner:
- The external area of the anus is cleaned as per usual after defecation.
- The cannula 2 is unsealed to open the orifices 6 where the solution for cleaning the internal anal canal will flow out.
- If the cannula 2 is independent from the deposit 1, the deposit 1 is unsealed and the cannula 2 is placed at the opening thereof made for this purpose by means of the suitable system (threading, pressure or another system).
- The cannula 2 is then inserted several centimeters into the external anal sphincter until reaching the region of the internal anal canal, to that end a type of lubricant can be used.
- Once the cannula 2 is inserted, the depressible deposit 1 is pressed such that the physiological cleaning solution is introduced in the anus in a variable amount sufficient for its cleaning and evacuation function.
- The amount of solution introduced with certain pressure removes the fecal matter from the walls of the internal anal canal and entrains the fecal matter remaining in said canal.
- The amount of solution introduced is sufficient to cause the natural evacuation of the solution through the anus due to the voluntary relaxation of the external anal sphincter.
- Together with the evacuation of the solution, fecal matter is eliminated from the internal anal canal, leaving the internal anal canal cleaner than with defecation alone.
- Finally, the perianal region is dried as per usual.

## Claims

1. Disposable device for internal anal canal hygiene, comprising an elongated cannula (2) connected to a deposit (1) refillable with a physiological solution, wherein the cannula (2) is provided with at least one orifice (6) in fluid communication with the deposit (1) and, wherein the deposit (1) is formed of a depressible material for pumping said physiological solution to the cannula (2) when pressure is exerted in said deposit (1).

2. Disposable device for internal anal canal hygiene, according to claim 1 wherein the cannula (2) and the deposit (1) are joined together forming a one single piece device.

3. Disposable device for internal anal canal hygiene, according to claim 1, wherein the cannula (2) is connected to the deposit (1) by means of screwed, pressure or piercing.

4. Disposable device for internal anal canal hygiene, according to any of the preceding claims, the cannula (2) having two ends, a proximal end (7), through which the cannula (2) is connected to the deposit (1) and, a free end (4), opposite to the proximal end (7), wherein the cannula (2) comprises a bellows system (3) at its proximal end (7).

5. Disposable device for internal anal canal hygiene, according to any of claims 1-3, wherein the deposit (1) comprises a bellows system (3) adjacent to its connection with the cannula (2).

6. Disposable device for internal anal canal hygiene, according to any of the preceding claims, the cannula (2) having two ends, a proximal end (7), through which the cannula (2) is connected to the deposit (1) and, a free end (4), opposite to the proximal end (7), wherein the free end (4) has a rounded tip (5) and, wherein the at least one orifice (6) is configured at its lateral sides.

7. Disposable device for internal anal canal hygiene, according to claim 6, wherein the device further comprises a seal to protect the perforated area of the free end (4) of the cannula (2).

8. Disposable device for internal anal canal hygiene, according to any of the preceding claims, wherein the cannula (2) is long enough to be inserted through the anal orifice and only reach the internal anal canal.

9. Disposable device for internal anal canal hygiene, according to any of the preceding claims, wherein the physiological solution is composed of a physiological entraining liquid to drag the rest of feces of the walls of the internal anal canal, such as physiological saline or purified water.

10. Disposable device for internal anal canal hygiene, according to claim 9, wherein the physiological solution further comprises menthol to provide freshness.

11. Disposable device for internal anal canal hygiene, according to any of claims 9-10, wherein the physiological solution further comprises organoleptic components, such as mint, strawberry, or rose essence.

12. Disposable device for internal anal canal hygiene, according to any of claims 9-11, wherein the physiological solution further comprises active principles for anal pathologies treatment.

13. Disposable device for internal anal canal hygiene, according to any of claims 9-12, wherein the physiological solution further comprises a vehicle component to solubilize the physiological solution, such as glycerin.
